# DEMANDE DE BREVET EUROPEEN

(11) **EP 0 562 936 A1**
(43) Date de publication de la demande: **29.09.1993**
(21) Numéro de dépôt: 93400728.7
(22) Date de dépôt: 22.03.1993
(51) Int. Cl.: C07D 235/02, A61K 31/415

(54) **Imidazolines N-substituées par un groupement biphénylméthyle, leur préparation, les compositions pharmaceutiques en contenant**

(30) Priorité: 23.03.1992 FR 9203470
(71) Demandeur: SANOFI, F-75008 Paris (FR)
(72) Inventeur: Ferrari, Bernard, F-34270 Les Matelles (FR); Arnaud-Taillades, Joelle, F-34080 Montpellier (FR)
(74) Mandataire: Gillard, Marie-Louise

(57) **Abrégé**

L'invention a pour objet des composés de formule: 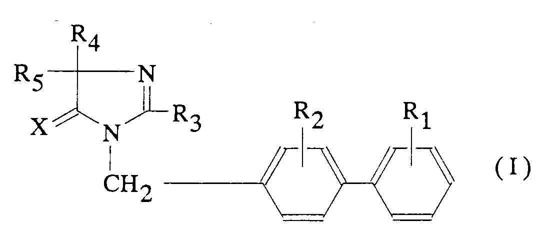
dans laquelle :
- R₁ et R₂ représentent chacun indépendamment H ou alkyle (C₁-C₆), alcoxy (C₁-C₄) , amino, aminométhyle, carboxy, alcoxycarbonyle, cyano, tétrazolyle, méthylsulfonylamino, trifluorométhylsulfonylamino, trifluorométhylsulfonylaminométhyle, N-cyano-acétamide, N-hydroxy-acétamide, N-((carboxy-4) thiazol-1,3-yl-2) acétamide, uréido, cyano-2 guanidinocarbonyle, cyano-2 guanidinométhyle, imidazol-1-yl-carbonyle, cyano-3 méthyl-2 isothioureidométhyle, à la condition qu'au moins l'un des substituants R₁ ou R₂ soit différent de l'hydrogène ;
- R₃ représente H, alkyle (C₁-C₆) , alcényle (C₂-C₆), cycloalkyle (C₃-C₇), phényle, phénylalkyle , phénylalcényle ;
- R₄ et R₅ représentent alkyle C₁-C₆, (C₃-C₇) cycloalkyle, phényle, phénylalkyle;
- ou R₄ et R₅ liés ensemble représentent, soit (CH₂)ₙ, soit (CH₂)ₚY(CH₂)_{q}, dans lequel Y est 0 ou S, soit C substitué par (C₁-C₄) alkyle , phényle ou phénylalkyle , soit N-R₆ où R₆ représente H, alkyle (C₁-C₄), phénylalkyle , alkylcarbonyle , halogéno-alkylcarbonyle, polyhalogénoalkylcarbonyle , benzoyle, α-aminoacyle ou un groupe N-protecteur, ou R₄ et R₅ ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un groupe N-R₇ ou un groupe C(CN) R₈ ;
- R₇ représente un alkyle en C₁-C₄ ou un groupe cyano ;
- R₈ représente H, cyano, tétrazolyle, *tert*-butyltétrazolyle, phénylsulfonyle ou un groupe COR₉ ;
- R₉ représente thiényle, furyle, alcoxy (C₁-C₄), phénoxy, benzyloxy ou phényle; et ses sels éventuels.

APPLICATION: antagonistes de l'angiotensine II.

## Description

La présente invention concerne des dérivés imidazolines N-substitués par un groupement biphénylméthyle, leur préparation et les compositions pharmaceutiques en contenant.

Les composés selon l'invention antagonisent l'action de l'angiotensine II qui est une hormone peptidique de formule:

H-Asp-Arg-Val-Tyr-Ile-His-Pro-Phe-OH

L'angiotensine II est un puissant vasopresseur qui est le produit biologiquement actif du système rénine-angiotensine : la rénine agit sur l'angiotensinogène du plasma pour produire l'angiotensine I, celle-ci est convertie en angiotensine II par action de l'enzyme de conversion de l'angiotensine I.

Les composés de la présente invention sont des composés non peptidiques, antagonistes de l'angiotensine II. En inhibant l'action de l'angiotensine II sur ses récepteurs, les composés selon l'invention empêchent notamment l'augmentation de la pression sanguine produite par l'interaction hormone-récepteur ; ils ont également d'autres actions physiologiques au niveau du système nerveux central.

Ainsi les composés selon l'invention sont utiles dans le traitement d'affections cardiovasculaires comme l'hypertension, la défaillance cardiaque ainsi que dans le traitement d'affections du système nerveux central et dans le traitement du glaucome et de la rétinopathie diabétique.

La demande de brevet européenne EP 454 511 décrit des composés de formule: 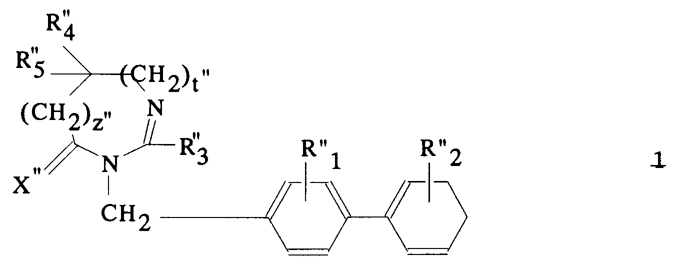
dans laquelle :
R''₁, R''₂, R''₃, R''₄, R''₅, z'' et t'' ont différentes valeurs et X'' représente un atome d'oxygène ou un atome de soufre.

Ces composés ont une activité antagoniste de l'angiotensine II.

La présente invention a pour objet des composés de formule: 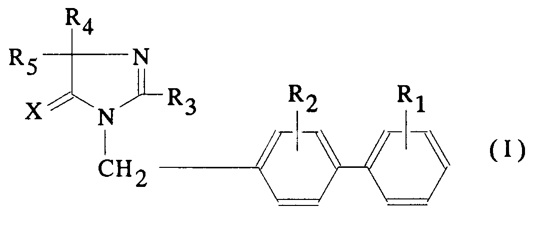
dans laquelle :
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol-1,3-yl-2) acétamide, un uréido, un cyano-2 guanidinocarbonyle, un cyano-2 guanidinométhyle, un imidazol-1-ylcarbonyle, un cyano-3 méthyl-2 isothiouréidométhyle, à la condition qu'au moins l'un des substituants R₁ ou R₂ soit différent de l'hydrogène ;
- R₃ représente un hydrogène, un alkyle en C₁-C₆ non substitué ou substitué par un ou plusieurs atomes d'halogène, un alcényle en C₂-C₆, un cycloalkyle en C₃-C₇ , un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un cycloalkyle en C₃-C₇, un phényle, un phénylalkyle dans lequel l'alkyle est en C₁-C₃, lesdits groupes alkyle, phényle ou phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄ , un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un α-aminoacyle ou un groupe N-protecteur, ou R₄ et R₅ liés ensemble avec l'atome de carbone auquel ils sont liés constituent un indane ou un adamantane ;
- p + q = m;
- n est un nombre entier compris entre 2 et 11 ;
- m est un nombre entier compris entre 2 et 5 ;
- X représente un groupe N-R₇ ou un groupe C(CN) R₈ ;
- R₇ représente un alkyle en C₁-C₄ ou un groupe cyano ;
- R₈ représente l'hydrogène, un groupe cyano, un groupe tétrazolyle, un groupe *tert*-butyltétrazolyle, un groupe phénylsulfonyle ou un groupe COR₉ ;
- R₉ représente un thiényle, un furyle, un alcoxy en C₁-C₄, un phénoxy, un benzyloxy ou un phényle non substitué ou substitué par un halogène, un alkyle en C₁-C₄ ou un polyhalogénoalkyle en C₁-C₄ , ;

et leurs sels éventuels.

Les composés de formule (I) dans lesquels :
- R₁ est en position ortho et représente un carboxyle ou un tétrazolyle ;
- R₂ est l'hydrogène ;
- R₃ représente un alkyle en C₁-C₆ ;
- R₄ et R₅ liés ensemble avec le carbone auquel ils sont attachés, représentent un groupe de formule (CH₂)ₙ;
- n est égal à 4 ou 5;
- X représente un groupe dicyanométhylène ;

et leurs sels, sont des composés préférés de l'invention.

Les sels éventuels des composés de formule (I) selon la présente invention comprennent ceux avec des acides minéraux ou organiques qui permettent une séparation ou une cristallisation convenable des composés de formule (I), tels que l'acide trifluoroacétique, l'acide picrique, l'acide oxalique ou un acide optiquement actif, par exemple un acide mandélique ou un acide camphosulfonique, et ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine pharmaceutiquement acceptable.

Selon la présente description et dans les revendications qui vont suivre, par atome d'halogène on entend un atome de brome, de chlore ou de fluor ; par groupe N-protecteur (également désigné par Pr) on entend un groupe utilisé classiquement dans la chimie des peptides pour permettre une protection temporaire de la fonction amine, par exemple un groupe Boc, Z, Fmoc ou un groupe benzyle ; par groupe carboxy estérifié on entend un ester labile dans des conditions appropriées, comme par exemple un ester méthylique, éthylique, benzylique ou tertiobutylique.

Le groupe α-aminoacyle représente le résidu d'un aminoacide naturel.

Les abréviations suivantes sont utilisées dans la description et dans les exemples
- Et: : Ethyl
- nBu, tBu: : n-butyl, *tert*-butyl
- DMF: : diméthylformamide
- THF: : tétrahydrofuranne
- DCM: : dichlorométhane
- DCC: : dicyclohexylcarbodiimide
- NBS: : N-bromosuccinimide
- DIPEA: : diisopropyléthylamine
- HOBT: : hydroxybenzotriazole
- MeOH: : méthanol
- AcOH: : acide acétique
- AcOEt: : acétate d'éthyle
- Ether: : éther éthylique
- TFA: : acide trifluoroacétique
- Z: : benzyloxycarbonyle
- Boc: : *tert*-butoxycarbonyle
- BOP: : hexafluorophosphate de benzotriazolyloxy trisdiméthylamino phosphonium
- Fmoc: : fluorénylméthyloxycarbonyle

La présente invention a également pour objet le procédé de préparation des composés (I). Ledit procédé est caractérisé en ce que :
a) on fait réagir un dérivé hétérocyclique de formule : 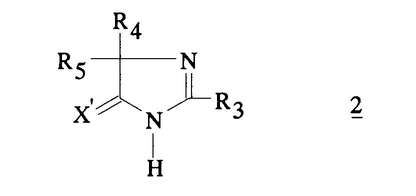 dans laquelle, R₃ , R₄, et R₅ ont les significations indiquées ci-dessus pour (I) et X' représente, soit un atome de soufre, soit X tel que défini pour (I), sur un dérivé du (biphényl-4-yl) méthyle de formule : 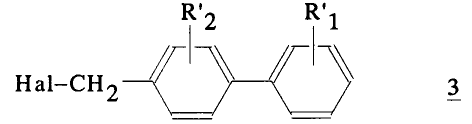 dans laquelle Hal représente un atome d'halogène et R'₁ et R'₂ représentent respectivement soit R₁ et R₂, soit un précurseur de R₁ et/ou R₂
b) éventuellement, le composé, ainsi obtenu de formule : 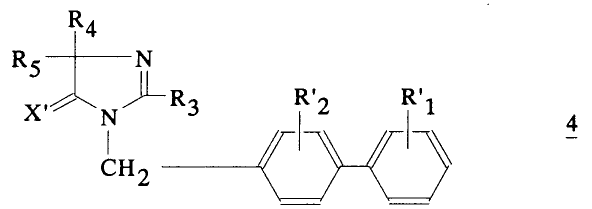 est traité pour préparer le composé (I) par transformation des groupes R'₁ et/ou R'₂ en respectivement R₁ et/ou R₂ et du groupe X' en X.
c) éventuellement le composé ainsi obtenu est transformé en l'un de ses sels.

Les composés 2 dans lesquels X' est X sont nouveaux et font partie de l'invention. Ainsi la présente invention a également pour objet les composés de formule : 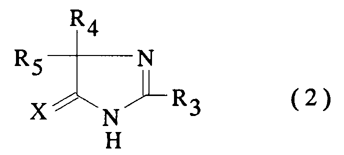
dans laquelle R₃, R₄ R₅ et X ont les valeurs données ci-dessus pour (I).

Les composés (2) sont préparés par des méthodes connues à partir des imidazoline-2 thione-5 correspondantes de formule : 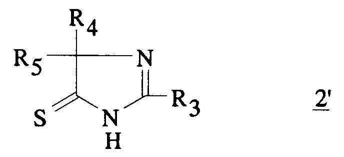
dans laquelle R₃, R₄ et R₅ ont les valeurs données ci-dessus pour (I).

La préparation des composés 2' peut être effectuée selon le procédé décrit dans la demande de brevet EP 454 511 : on peut utiliser la méthode décrite par Jacquier et al. (Bull. Soc. Chim France, 1971, (3), 1040-1051) et faire agir un imidate d'alkyle 6 sur un acide aminé ou son ester 5; le composé 7 ainsi obtenu est alors traité par le réactif de Lawesson [bis (méthoxy-4 phényl)-2,4 dithia- 1,3 disphosphétane-2,4 disulfure-2,4], selon le schéma réactionnel suivant : 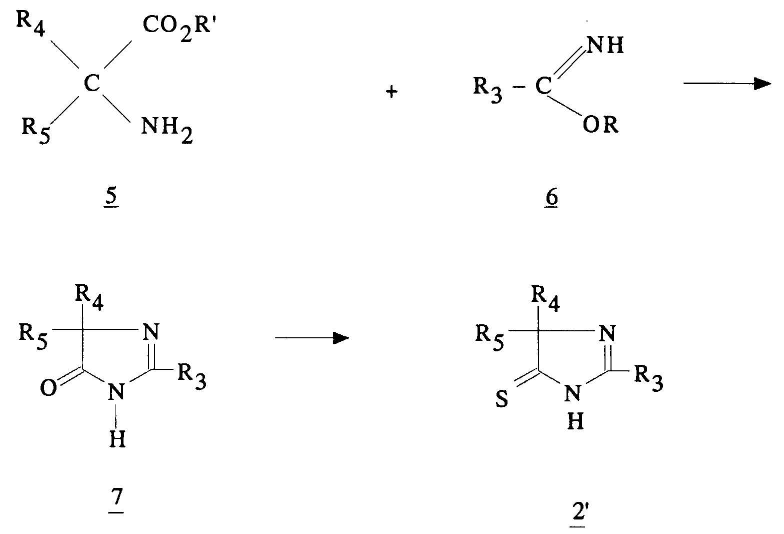
dans lequel R représente un alkyle en C₁-C₄ et R' représente l'hydrogène ou un alkyle en C₁-C₄, R₃, R₄ et R₅ sont tels que définis précédemment.

On peut également faire agir sur un composé 5, dans lequel R' est un groupe amino, un chlorure d'acide (6') R₃COCl et préparer ainsi le composé 7.

Lorsque R₄ et R₅ sont différents, les composés 2' peuvent être obtenus optiquement purs en utilisant des méthodes de synthèse asymétrique ou des méthodes de résolution du mélange racémique telles que décrites dans Synthesis of Optically Active, α-aminoacids, R.M. Williams, Pergamon Press. 1989.

Différents procédés utilisables pour la préparation d'un composé (2) à partir d'un composé 2' sont rapportés ci-après

La préparation des composés de formule (2) selon l'invention, dans lesquels X représente un groupement imine non substitué ou substitué par un alkyle en C₁-C₄ ou par un groupe cyano, est effectuée selon J. Marchand-Brynaert dans J. Chem. Soc. Chem. Commun., 1983, 818: on fait agir sur un composé de formule 2' par exemple, une amine primaire portant le substituant désiré par exemple en présence de diacétate de mercure.

La préparation des composés de formule (I) selon l'invention, dans lesquels X représente un groupe imine substitué par un alkyle en C₁-C₄ ou par un groupe cyano, est préférentiellement effectuée à partir de composés 4 dans lesquels X' représente un atome de soufre ; la transformation d'un composé 4 en un composé (I) selon l'invention est effectuée selon la méthode de J. Marchand Brynaert, décrite ci-dessus.

La préparation des composés (2) selon l'invention, dans lesquels X représente un groupe cyanométhylène substitué par un autre cyano ou par un groupe COR₉, est effectuée en utilisant le procédé décrit par Z.T. Huang et al., dans Chem. Ber., 1968, 119, 2-2219 : on fait agir un composé de formule CN-CH₂-CN (8) ou CN-CH₂-COR₉(8') sur un dérivé méthylthio de l'imidazole de formule

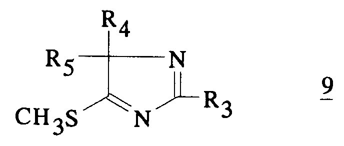

Selon les mêmes auteurs, la préparation des composés de formule (2) selon l'invention dans lesquels X représente un groupe cyanométhylène substitué par un phénylsulfonyle peut être effectuée par action d'un composé de formule

CN-CH₂-SO₂-C₆H₅

sur un composé de formule 9.

Le composé de formule 9 est lui-même obtenu par action de l'iodure de méthyle sur l'imidazoline-2 thione-5 (2') correspondante.

De plus, les composés (I), dans lesquels X représente un groupe dicyanométhylène, peuvent être préparés selon Y. Tominaga et al., dans Heterocycles, 1987, 26(3), 613 : on fait agir l'oxyde de tétracyanoéthylène sur un groupement thione. La réaction peut être effectuée, soit sur un composé (I), dans lequel X est remplacé par un groupement thione, soit sur un imidazoline-2 thione-5 (2').

Les composés (2) selon l'invention, dans lesquels X représente un groupe C(CN)R₈, R₈ étant un tétrazolyle ou un *tert*-butyltétrazolyle, sont préparés à partir des composés (2) correspondants, dans lesquels R₈ est un groupe cyano, par action d'un azide, par exemple l'azide de tributylétain.

Les composés de formule (2) selon l'invention dans lesquels X représente un groupe -CH-CN sont préparés à partir des composés de formule (2) dans lesquels X représente -C(CN)CO₂Alk, Alk représentant un alkyle en C₁-C₄, par action d'un acide fort, tel que l'acide trifluoracétique.

Le dérivé du (biphényl-4-yl) méthyle (3) est préparé par un procédé dérivé de celui décrit dans la demande de brevet européen 324 377.

Ainsi la préparation d'un composé 3 dans lequel R'₁ et/ou R'₂ représente un groupe carboxy ou carboxyester est décrite dans la demande de brevet citée ci-dessus.

On utilise les méthodes connues de la littérature pour préparer des composés 3 dans lesquels R₁ et/ou R₂ ont l'une des valeurs décrites ci-dessus pour (I). Ces méthodes sont décrites dans la demande EP 454511. L'obtention du groupement R₁ et/ou R₂ souhaité peut également être effectuée à la dernière étape du procédé décrit ci-dessus à partir du composé 4 dans lequel R'₁ et/ou R'₂ représente un groupe carboxy ou un dérivé d'un groupe carboxy. Un tel composé est préparé à partir d'un composé 3 dans lequel R'₁ et/ou R'₂ représente un groupe permettant de préparer R₁ et/ou R₂.

Dans le procédé selon l'invention, l'étape a) est réalisée dans un solvant inerte tel que le DMF, le DMSO ou le THF, en milieu basique, par exemple en présence de potasse de soude, de carbonate de potassium, d'un alcoolate métallique, d'un hydrure métallique ou de triéthylamine.

Les composés de formule (I) selon l'invention, dans lesquels R₄ et R₅ liés ensemble représentent un groupe de formule (CH₂)ₚY(CH₂)_{q} dans lequel Y est un groupe NH, peuvent être préparés par hydrogénation catalytique d'un composé de formule (I) correspondant dans lequel Y est un groupe N-R₆, R₆ étant un benzyle.

L'affinité des produits selon l'invention pour les récepteurs de l'angiotensine II a été étudiée sur un test de liaison de l'angiotensine II marquée à l'iode 125 à des récepteurs membranaires de foie de rats. La méthode utilisée est celle décrite par S. Keppens et al. dans Biochem. J., 1982, 208, 809-817.

On mesure la CI₅₀ : concentration qui donne 50% de déplacement de l'angiotensine II marquée, liée spécifiquement au récepteur. La CI₅₀ des composés selon l'invention est inférieure à 10⁻⁶M.

De plus l'effet antagoniste de l'angiotensine II des produits selon l'invention a été constaté sur différentes espèces animales dans lesquelles le système rénine-angiotensine a été préalablement activé (C. Lacour et al., J. Hypertension, 1989, 7 (suppl.2), S33-S35).

Les composés selon l'invention sont actifs après administration par différentes voies, notamment par voie orale.

Aucun signe de toxicité n'est observé pour ces composés aux doses pharmacologiquement actives.

Ainsi les composés selon l'invention peuvent être utilisés dans le traitement de différentes affections cardiovasculaires, notamment l'hypertension, la défaillance cardiaque, les insuffisances veineuses, ainsi que dans le traitement du glaucome, des rétinopathies diabétiques et de différentes affections du système nerveux central, l'anxiété, la dépression, les déficits mnésiques ou la maladie d'Alzheimer par exemple.

La présente invention a également pour objet des compositions pharmaceutiques contenant une dose efficace d'un composé selon l'invention ou d'un sel pharmaceutiquement acceptable et des excipients convenables. Lesdits excipients sont choisis selon la forme pharmaceutique et le mode d'administration souhaités.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule (I) ci-dessus, ou leurs sels éventuels, peuvent être administrés sous forme unitaire d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres,les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un de leurs sels pharmaceutiquement acceptables, d'autres principes actifs tels que, par exemple, des tranquillisants ou d'autres médicaments qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention et le ou les autres peuvent être un composé béta-bloquant, un antagoniste calcique, un diurétique, un antiinflammatoire non stéroïdien ou un tranquillisant.

Les exemples suivants illustrent l'invention sans toutefois la limiter. Dans ces exemples, on utilise les abréviations suivantes :

TA signifie température ambiante, KHSO₄-K₂SO₄ signifie une solution aqueuse contenant 16,6 g de bisulfate de potassium et 33,3 g de sulfate de potassium pour 1 litre. D'une façon générale, les solutions salines employées sont des solutions aqueuses.

Les points de fusion (Fc) sont donnés en degrés Celsius ; sauf indication contraire, ils sont mesurés sans recristallisation du produit.

La pureté des produits est vérifiée en chromatographie sur couche mince (CCM) ou par CLHP (chromatographie liquide à haute performance). Les produits sont caractérisés par leur spectre RMN enregistré à 200 MHz dans le DMSO deutérié, la référence interne étant le tétraméthylsilane, sauf indication contraire.

Pour l'interprétation des spectres de RMN, on emploie :
- s: pour singulet
- s.e.: pour singulet élargi
- d: pour doublet
- t: pour triplet
- q: pour quadruplet
- quint: pour quintuplet
- sext: pour sextuplet
- m: pour massif ou multiplet

Les composés de formule (I) selon l'invention, dans lesquels X représente un groupe C(CN) R₈, R₈ étant tel que défini ci-dessus pour (I), présentent, lorsque R₈ est autre que CN, une isomérie cis-trans autour de la liaison méthylène.

On peut observer généralement la présence des 2 isomères en équilibre ; cependant, pour certaines valeurs de R₈, l'une des formes peut être prépondérante.

L'étude des isomères peut être faite, soit par coalescence en RMN à haute température, soit par RMN 1 H N.O.E.S.Y. (Nuclear Overhauser Effect Spectroscopy) à 2 dimensions. Un tel phénomène a été décrit pour les énaminocétones et énaminoacides par O. Michinori, dans Methods in Stereochemical Analysis of Dynamic NMR Spectroscopy in Organic Chemistry.

### EXEMPLE 1

Trifluoroacétate de N-isopropyl n-butyl-2[(carboxy-2'biphényl-4-yl) méthyl]-1 spirocyclopentane-4 imidazoline-2 imine-5.

(I, X = = NCH(CH₃)₂)

Ce composé est préparé à partir de la n-butyl-2 spirocyclopentane-4 [(*tert*-butoxycarbonyl-2' biphényl-4-yl) méthyl]-1 imidazoline-2 thione-5. Cette imidazoline-2 thione-5, ci-après dénommée composé A, est décrite dans la demande de brevet EP 454511.

La transformation de la thione en N-isopropyl imine est effectuée selon J. Marchand-Brynaert et al. dans J. Chem. Soc. Chem. Commun., 1983, 818.

On traite 150 mg d'imidazoline-2 thione-5 (composé A) en solution dans 4 ml de DCM par 1 ml d'isopropylamine en présence de diacétate de mercure et on laisse sous agitation pendant 5 jours à TA. Après filtration sur Célite ® , le filtrat est concentré et le résidu est repris par 50 ml d'acétate d'éthyle. Après lavage par 20 ml d'une solution saturée de chlorure de sodium, séchage sur sulfate de sodium et concentration, le résidu est purifié par chromatographie sur silice en éluant par un mélange toluène/acétate d'éthyle (3/1, v/v). L'huile obtenue (50 mg) est traitée par un mélange TFA/DCM (3 ml/3 ml) pendant 1 heure à TA pour donner le composé attendu sous forme d'une huile claire.
- Spectre de masse : MH⁺ : 446
- Spectre RMN:
   0,8 ppm : t : 3 H : CH₃(nBu)
   1 et 1,1 ppm : 2 d : 6 H : (CH₃)₂ CHN=
   1,2-2,2 ppm : m : 12 H : cyclopentane et CH₃-CH₂-CH₂-CH₂-
   2,45 ppm : t : 2 H : CH₃-CH₂-CH₂-CH₂-
   3,9 ppm : m : 1 H : (CH₃)₂ CH-N=
   5,05 ppm : s : CH₂-C₆H₄-
   6,9-8,1 ppm : m : 8 H : aromatiques

Le spectre RMN indique la présence d'un mélange d'isomères syn et anti du groupe N(isopropyl) imine.

### EXEMPLE 2

Trifluoroacétate de n-butyl-2 [(carboxy-2' biphényl-4-yl) méthyl]-1 dicyanométhylène-5 spirocyclopentane-4 imidazoline-2.

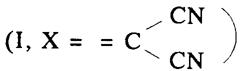

### A) n-butyl-2 dicyanométhylène-5 spirocyclopentane-4 [(tert-butoxycarbonyl-2'biphényl-4-yl) méthyl]-1 imidazoline-2.

On traite 518 mg du composé A décrit à l'exemple 1 en solution dans 17 ml de benzène anhydre par 156 mg d'oxyde de tétracyanoéthylène, pendant 5 heures, à TA, sous argon. Le milieu réactionnel est concentré et chromatographié sur silice en éluant par un mélange toluène-AcOEt (97/3, v/v). On obtient 200 mg du produit attendu sous forme huileuse.
- Spectre RMN :
1,0 ppm: t: 3 H: CH₃(nBu)
1,35 ppm: s: 9 H: tBu
1,5 ppm : sext: 2 H: CH₃-CH₂-CH₂-CH₂-
1,8 ppm: quint: 2H: CH₃-CH₂-CH₂-CH₂-
1,9-2,25 ppm : m : 8 H: cyclopentane
2,55 ppm : t : 2 H: CH₃-CH₂-CH₂-CH₂-
5,4 ppm : s : 2 H : CH₂-C₆H₄-
7,15-7,95 ppm : m : 8 H: aromatiques

### B) Trifluoroacétate de n-butyl-2[(carboxy-2' biphényl-4-yl) méthyl]-1 dicyanométhylène-5 spirocyclopentane-4 imidazoline-2.

180 mg de l'huile obtenue sont traités par un mélange DCM-TFA (10ml/10 ml) pendant 1 heure à TA. Après concentration, le résidu est trituré dans un mélange éther-hexane. Le solide jaune obtenu est essoré et séché sous vide.
m = 170 mg
- Spectre de masse MH⁺ : 453
- Spectre RMN :
   0,8 ppm : t : 3 H : CH₃(nBu)
   1,25 ppm : sext : 2 H : CH₃-CH₂-CH₂-CH₂-
   1,5 ppm : quint : 2 H : CH₃-CH₂-CH₂-CH₂-
   1,8-2,5 ppm : m : 10 H : cyclopentane + CH₃-CH₂-CH₂-CH₂-
   5,3 ppm : s: 2 H : CH₂-C₆H₄-
   7,05-7,7 ppm : m : 8 H: aromatiques

### EXEMPLE 3

n-butyl-2 dicyanométhylène-5 spirocyclopentane-4 [((tétrazolyl-5)-2'biphényl-4-yl) méthyl]-1 imidazoline-2.

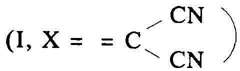

### A) n-butyl-2 spirocyclopentane-4 imidazoline-2 thione-5.

Ce composé est préparé à partir de la n-butyl-2 spirocyclopentane-4 imidazoline-2 one-5 décrit dans la demande EP 454511, par action du réactif de Lawesson [bis(méthoxy-4 phényl)-2,4 dithia-1,3 diphosphétane-2,4 disulfure-2,4].

7,58 g d'imidazoline-2 one-5, sous forme de base, sont dissous dans 50 ml de toluène, sous azote. On ajoute 7,90 g de réactif de Lawesson et on porte à reflux pendant 2 heures. On concentre à sec, le résidu est purifié par chromatographie sur silice en éluant par un mélange DCM-AcOEt (9/1, v/v). On obtient 5,95 g du produit attendu.
Fc = 96°C
- Spectre RMN :
0,8 ppm : t : 3 H : CH₃(nBu)
1,2 ppm : sext : 2 H : CH₃-CH₂-CH₂-CH₂-
1,4-1,8 ppm : m : 10 H : cyclopentane + CH₃-CH₂-CH₂-CH₂-
2,35 ppm : t : 2 H : CH₃-CH₂-CH₂-CH₂-
5,3 ppm : s : 1H : NH

### B) n-butyl-2 dicyanométhylène-5 spirocyclopentane-4 imidazoline-2

1 g du composé préparé à l'étape précédente est dissous dans 50 ml de benzène anhydre, sous azote. On ajoute 686 mg d'oxyde de tétracyanoéthylène et laisse 1 heure sous agitation à TA. Le précipité formé est essoré puis séché sous vide et purifié par chromatographie sur silice en éluant par un mélange heptane-acétate d'éthyle (2/8, v/v). Le produit obtenu est trituré dans l'éther puis filtré.
m = 510 mg
Fc = 145°C
- Spectre RMN :
1 ppm : t : 3H : CH₃(nBu)
1,2 ppm : sext : 2 H : CH₃-CH₂-CH₂-CH₂-
1,7 ppm : quint : 2 H : CH₃-CH₂-CH₂-CH₂-
1,8-2,4 ppm : m : 8 H : cyclopentane
2,6 ppm : t : 2 H: CH₃-CH₂-CH₂-CH₂-
11,4 ppm : s : 1 H : NH

### C) n-butyl-2 dicyanométhylène-5 spirocyclopentane-4 [((trityltétrazolyl-5)-2' biphényl-4-yl) méthyl]-1 imidazoline-2.

On place sous azote un mélange contenant 70 mg d'hydrure de sodium à 80% dans l'huile et 10 ml de DMF et l'on ajoute goutte à goutte 500 mg du composé préparé à l'étape B), dissous dans 10 ml de DMF anhydre. Après 20 minutes d'agitation à TA, on ajoute 1,2 g de bromométhyl-4(triphénylméthyltétrazolyl-5)-2' biphényle et on agite pendant 4 heures à 40°C sous azote. Après concentration du milieu, on reprend le résidu par AcOEt. La phase organique est lavée par de l'eau, une solution saturée de ClNa, une solution KHSO₄-K₂SO₄ à 5%, puis une solution saturée de ClNa. On sèche et concentre puis on purifie l'huile obtenue par chromatographie sur silice en éluant par un mélange heptane-acétate d'éthyle (7/3, v/v). Le produit obtenu cristallise dans l'acétate d'éthyle.
m = 700 mg

### D) n-butyl-2 dicyanométhylène-5 spirocyclopentane-4 [((tétrazolyl-5)-2' biphényl-4-yl) méthyl]-1 imidazoline-2.

On place 700 mg de produit obtenu à l'étape précédence dans 10 ml d'acide acétique à 5% dans le méthanol et on porte à reflux pendant 24 heures. On concentre à sec puis le résidu est purifié par chromatographie sur silice en éluant par un mélange DCM/AcOEt/AcOH (90/10/10). Le produit obtenu est trituré dans l'éther ; le précipité formé est filtré et séché sous vide.
m = 180 mg
Fc = 181°C
- Spectre de masse : MH⁺ = 477
- Spectre RMN :
   0,8 ppm : t : 3 H : CH₃(nBu)
   1,25 ppm : sext : 2 H : CH₃-CH₂-CH₂-CH₂-
   1,5 ppm : quint : 2 H : CH₃-CH₂-CH₂-CH₂-
   1,8-2,4 ppm : m : 10 H : cyclopentane + CH₃-CH₂-CH₂-CH₂-
   5,3 ppm : s : 2 H : CH₂-C₆H₄-
   7,0-7,8 ppm : m : 8 H : aromatiques

### EXEMPLE 4

n-butyl-2 (cyano-1 éthoxycarbonyl-1 méthylène)-5 [(carboxy-2' biphényl-4-yl) méthyl]-1 spirocyclopentane-4 imidazoline-2.

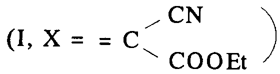

### A) n-butyl-2 méthylthio-5 spirocyclopentane-4 imidazole.

On place 3 g de n-butyl-2 spirocyclopentane-4 imidazoline-2 thione-5, décrit à l'exemple 3, étape A), dans 50 ml de THF anhydre, sous azote et on ajoute par petites quantités, 470 mg d'hydrure de sodium à 80% dans l'huile. Après 20 minutes d'agitation à TA, sous azote, on ajoute 900 µl d'iodure de méthyle et on laisse l'agitation sous azote pendant 4 heures. On ajoute 20 ml d'eau puis on concentre le milieu réactionnel. L'huile obtenue est reprise par 100 ml d'acétate d'éthyle puis lavée par de l'eau, une solution saturée de NaCl, une solution saturée de NaHCO₃, une solution saturée de NaCl ;on sèche sur sulfate de sodium et concentre.
m = 3,2 g
- Spectre RMN :
1 ppm : t : 3 H : CH₃(nBu)
1,4 ppm : sext : 2 H : CH₃-CH₂-CH₂-CH₂-
1,6-2 ppm : m : 10 H : cyclopentane + CH₃-CH₂-CH₂-CH₂-
2,6 ppm : t : 2 H : CH₃-CH₂-CH₂-CH₂-
2,65 ppm : s : 3 H: SCH₃-

### B) n-butyl-2 (cyano-1 éthoxycarbonyl-1 méthylène)-5 spirocyclopentane-4 imidazoline-2.

On chauffe à 100°C, pendant 6 heures, un mélange contenant 1 g du produit préparé à l'étape précédente et 476 µl de cyanoacétate d'éthyle. L'huile obtenue est purifiée par chromatographie sur silice en éluant par le mélange DCM-acétone (100/1, v/v).
m=1 g
- Spectre RMN :
0,85 ppm : t : 3 H : CH₃(nBu)
1,15-1,4 ppm : m : 5 H : CH₃ (Et) + CH₃-CH₂-CH₂-CH₂-
1,45-2,3 ppm : m : 10 H : cyclopentane + CH₃-CH₂-CH₂-CH₂-
2,45 ppm : t : 2 H : CH₃-CH₂-CH₂-CH₂-
4,2 ppm : q : 2 H : CH₂(Et)
11,4 ppm : s : 1H :NH

### C) n-butyl-2 (cyano-1 éthoxycarbonyl-1 méthylène)-5 spirocyclopentane-4 [(tert-butoxycarbonyl-2' biphényl-4-yl) méthyl]-1 imidazoline-2.

On place sous argon 114 mg d'hydrure de sodium à 80% dans l'huile, en suspension dans 5 ml de DMF anhydre. On ajoute 1 g du composé préparé à l'étape B dans 5 ml de DMF anhydre et l'on agite pendant 20 minutes, à TA, sous azote. On ajoute 1,32 g de bromométhyl-4 *tert*-butoxycarbonyl-2' biphényle et l'on agite sous azote pendant 2 heures à TA et pendant 4 heures à 60°C. On concentre, reprend le résidu par 50 ml d'acétate d'éthyle, puis lave la phase organique par de l'eau, une solution KHSO₄-K₂SO₄ à 5%, une solution saturée de NaCl, une solution saturée de NaHCO₃, une solution saturée de NaCl. On sèche et concentre, puis on purifie l'huile obtenue par chromatographie sur silice en éluant par un mélange heptane-AcOEt (9/1, v/v).
m = 860 mg

### D) n-butyl-2(cyano-1 éthoxycarbonyl-1 méthylène)-5 [(carboxy-2' biphényl-4-yl) méthyl]-1 spirocyclopentane-4 imidazoline-2.

800 g du produit obtenu à l'étape précédente sont dissous dans 10 ml de mélange TFA-DCM (5/5, v/v) et laissés sous agitation à TA, pendant 3 heures. On concentre à sec puis on co-évapore l'huile obtenue 2 fois avec de l'éther. L'huile est ensuite triturée dans de l'éther, on filtre le précipité formé et on le sèche sous vide. Le résidu est purifié par chromatographie sur silice en éluant par un mélange DCM-AcOEt-AcOH (90/10/2, v/v/v).
m = 240 mg
Fc = 102°C
- Spectre de masse : MH⁺ : 500,2
- Spectre RMN :
   0,9 ppm : t : 3 H : CH₃(nBu)
   1,2-2,8 ppm : m : 17 H : cyclopentane + CH₃ (Et) + CH₃-CH₂-CH₂-CH₂-
   4,2 ppm : m : 2 H : -OCH₂-CH₃
   5,3 et 5,6 ppm : 2 s: 2 H: CH₂-C₆H₄-
   7-7,9 ppm : m : 8 H: aromatiques

Le spectre RMN indique que les formes cis et trans du méthylène sont en équilibre.

### EXEMPLES 5 et 6

n-butyl-2 [(carboxy-2' biphényl-4-yl) méthyl]-1(cyano-1 *tert*-butoxycarbonyl-1 méthylène)-5 spirocyclopentane-4 imidazoline-2.

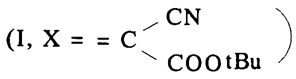

n-butyl-2 [(carboxy-2' biphényl-4-yl) méthyl]-1(cyano-1 méthylène)-5 spirocyclopentane-4 imidazoline-2.

(I, X = = CHCN).

### A) n-butyl-2(cyano-1 tert-butoxycarbonyl-1 méthylène)-5 spirocyclopentane-4 imidazoline-2.

Ce composé est préparé selon le procédé décrit à l'exemple 4, étape B), en faisant agir le cyanoacétate de *tert*-butyle sur le composé préparé à l'exemple 4, étape A)
- Spectre RMN :
0,85 ppm : t : 3 H : CH₃(nBu)
1,35 ppm : sext : 2 H : CH₃-CH₂-CH₂-CH₂-
1,45-2,35 ppm : m : 19 H : tBu + cyclopentane + CH₃-CH₂-CH₂-CH₂-
2,5 ppm : t : 2 H : CH₃-CH₂-CH₂-CH₂-
11,4 pp : s : 1 H : NH

### B) n-butyl-2(cyano-1 tert-butoxycarbonyl-1 méthylène)-5 spirocyclopentane-4 [(tert-butoxycarbonyl-2' biphényl-4-yl) méthyl]-1 imidazoline-2.

Ce composé est préparé selon le procédé décrit à l'exemple 4, étape C).
- Spectre RMN :
0,8 ppm : m : 3 H: CH₃(nBu)
1-2,6 ppm : m : 32 H : 2 tBu + cyclopentane + CH₃-CH₂-CH₂-CH₂-
5,3 et 5,45 ppm : 2 s : 2 H : CH₂-C₆H₄-
6,95-7,7 ppm : m : 8 H : aromatiques

### C) n-butyl-2[(carboxy-2' biphényl-4-yl) méthyl]-1 (cyano-1 tert-butoxycarbonyl-1 méthylène)-5 spirocyclopentane-4 imidazoline-2.

Ce composé est obtenu par action du TFA sur le composé décrit à l'exemple 4, étape D). L'huile obtenue est purifiée par chromatographie sur silice en éluant par un mélange heptane/AcOEt/AcOH (60/40/1,5, v/v/v). On sépare 2 produits.
1er produit :
- Spectre de masse : MH⁺ = 528
- Spectre RMN :
   0,8 ppm : m : 3 H: CH₃(nBu)
   1,2-2,5 ppm : m : 14 H : CH₃-CH₂-CH₂-CH₂- + cyclopentane
   5,3 et 5,4 ppm : 2 s : 2 H : CH₂-C₆H₄-
   6,9-7,8 ppm : m : 8 H : aromatiques

Le spectre RMN (2 s à 5,3 et 5,4 ppm) indique la présence de 2 formes cis et trans du méthylène, en équilibre.
2ème produit
- Spectre de masse : MH⁺ = 428
- Spectre RMN :
   0,8 ppm : m : 3 H: CH₃(nBu)
   1,2-2,4 ppm : m: 14 H : CH₃-CH₂-CH₂-CH₂- + cyclopentane
   4,6 et 4,8 ppm : 2 s : 2 H : CH₂-C₆H₄-
   6,9-7,7 ppm : m : 9 H : aromatiques + = CHCN

Le spectre RMN indique la présence des 2 formes cis et trans du méthylène.

### EXEMPLES 7 et 8

Hemiacétate de n-butyl-2[(carboxy-2' biphényl-4-yl) méthyl]-1 (cyano-1 (*tert*-butyltétrazol-5-yl)-1 méthylène)-5 spirocyclopentane-4 imidazoline-2. 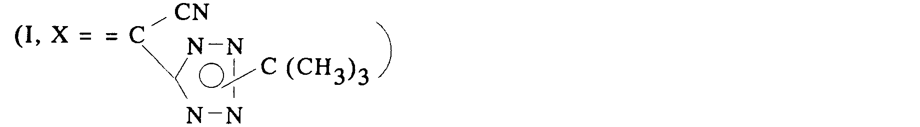
et n-butyl-2[(carboxy-2' biphényl-4-yl) méthyl]-1 (cyano-1 (tétrazol-5-yl)-1 méthylène)-5 spirocyclopentane-4 imidazoline-2

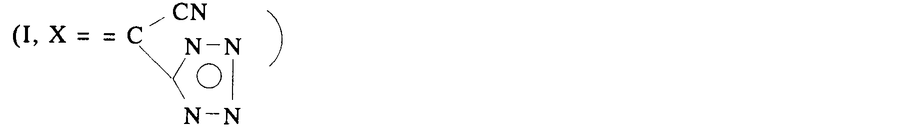

### A) n-butyl-2(cyano-1 (tétrazol-5-yl)-1 méthylène)-5 spirocyclopentane-4 [(tert-butoxycarbonyl-2' biphényl-4-yl) méthyl]-1 imidazoline-2

On place 740 mg du composé, préparé à l'exemple 2, étape A), dans 10 ml de xylène et on traite par 1,5 g d'azide de tributylétain, pendant 48 heures à reflux. Après concentration, le milieu est repris par un mélange éther-soude à 5% (20/20, v/v) et on laisse sous agitation pendant 15 minutes à TA. La phase aqueuse est décantée et la phase éthérée est extraite par 30 ml de soude à 5%. Les phases aqueuses sont lavées par de l'éther, puis acidifiées jusqu'à pH2 par addition d'acide chlorhydrique concentré. La gomme marron obtenue est extraite par 100 ml d'AcOEt. La phase organique est lavée par une solution saturée de chlorure de sodium, puis séchée sur sulfate de sodium. Après filtration et concentration, la mousse marron obtenue est purifiée par chromatographie sur silice en éluant par un mélange heptane-acétone-AcOH (80/20/1, v/v/v).

On obtient le produit attendu sous forme d'une huile.
m = 450 mg
- IR (DCM): 2200 cm⁻¹ (CN)
- Spectre RMN :
   0,8 ppm : m : 3 H : CH₃(nBu)
   1-2,4 ppm : m : 23 H : tBu + cyclopentane + CH₂-CH₂-CH₂- CH₃
   4,9 et 5,4 ppm : 2 s : CH₂-C₆H₄-
   6,4-7,6 ppm : m : 8 H : aromatiques

### B) Hémiacétate de n-butyl-2[(carboxy-2' biphényl-4-yl) méthyl]-1 (cyano-1 (tert-butyltétrazol-5-yl)-1 méthylène)-5 spirocyclopentane-4 imidazoline-2

400 mg du composé obtenu à l'étape précédente sont dissous dans un mélange DCM-TFA (5/10, v/v) et laissés sous agitation pendant 3 heures à TA. Après concentration à sec, le résidu est trituré dans un mélange éther-hexane (1/9, v/v), la phase solide obtenue est essorée et séchée sous vide. On purifie par chromatographie sur silice en éluant par un mélange DCM-MeOH-AcOH (100/2/1, v/v/v). On isole un premier composé. Celui-ci est trituré dans un mélange éther-hexane (1/9, v/v) puis essoré. On obtient le produit attendu sous forme d'une poudre blanche.
m = 160 mg
Fc = 210-212°C
- Spectre RMN :
0,75 ppm : t : 3 H : CH₃(nBu)
1,2 ppm : sext : 2 H: CH₃-CH₂-CH₂-CH₂-
1,4-2,4 ppm : m : 21 H : tBu + cyclopentane + CH₃-CH₂-CH₂-CH₂-
4,8 et 5,3 ppm : 2 s : 2 H : -CH₂-C₆H₄-
6,4-7,6 ppm : m : 8 H : aromatiques

### C) n-butyl-2[(carboxy-2' biphényl-4-yl) méthyl]-1 (cyano-1 (tétrazol-5-yl)-1 méthylène)-5 spirocyclopentane-4 imidazoline-2

La chromatographie effectuée à l'étape B) permet d'isoler un deuxième composé. Celui-ci est obtenu sous forme d'une poudre blanche après trituration dans un mélange éther-hexane (1/1, v/v) et essorage.
m = 220 mg
Fc = 217-219°C
- Spectre RMN :
0,85 ppm : t : 3H : CH₃(nBu)
1,3 ppm : sext : 2 H : CH₃-CH₂-CH₂-CH₂-
1,6 ppm : quint : 2 H : CH₃-CH₂-CH₂-CH₂-
1,8-2,6 ppm : m : 10 H cyclopentane + CH₃-CH₂-CH₂-CH₂-
4,9 et 5,4 ppm : 2 s : 2 H : -CH₂-C₆H₄-
6,4-7,7 ppm : m : 8 H : aromatiques
12,7 ppm : s : 1 H : CO₂H

Les spectres RMN des exemples 7 et 8 montrent l'existence des formes cis et trans du méthylène en équilibre. Pour le composé de l'exemple 7, la position du groupe t-butyle sur le tétrazole n'est pas déterminée; la formule développée représentée ci-dessus est donc arbitraire.

### EXEMPLE 9

Trifluoroacétate de (benzoyl-1 cyano-1 méthylène)-5 n-butyl-2 [(carboxy-2' biphényl-4-yl) méthyl]-1 spirocyclopentane-4 imidazoline-2.

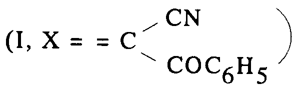

### A) (benzoyl-1 cyano-1 méthylène)-5 n-butyl-2 spirocyclopentane-4 imidazoline-2.

On dissout dans le minimum de DCM 2 g du composé préparé à l'exemple 4, étape A) et 1,30 g de benzoylacétonitrile et l'on chauffe à 100°C, sous agitation, pendant 8 heures. L'huile obtenue est chromatographiée sur silice en éluant par un mélange heptane-AcOEt (8/2, v/v). On obtient 1,63 g du produit attendu.
- Spectre RMN :
0,95 ppm : t : 3 H : CH₃(nBu)
1,4 ppm : sext : 2 H : CH₃-CH₂-CH₂-CH₂-
1,65 ppm : quint : 2 H : CH₃-CH₂-CH₂-CH₂-
1,7-2,7 ppm : m : 10 H cyclopentane + CH₃-CH₂-CH₂-CH₂-
7,4-8,0 ppm : m : J H : aromatiques
12,4 ppm : s : 1 H :NH

### B) (benzoyl-1 cyano-1 méthylène)-5 n-butyl-2 spirocyclopentane-4 [(tert-butoxycarbonyl-2' biphényl-4-yl) méthyl]-1 imidazoline-2.

On dissout dans 20 ml de DMF anhydre sous azote, 800 mg de l'imidazoline préparée à l'étape précédente, 700 mg de carbonate de potassium et 1,8 g de bromométhyl-4 *tert*-butoxycarbonyl-2' biphényle. On chauffe le milieu réactionnel à 70°C, sous agitation, pendant 5 heures. On extrait par AcOEt, lave la phase organique par de l'eau, puis par une solution saturée de chlorure de sodium. Le produit obtenu est chromatographié sur silice en éluant par un mélange heptane-AcOEt (8/2, v/v). On obtient 820 mg du produit attendu.

### C) Trifluoroacétate de (benzoyl-1 cyano-1 méthylène)-5 n-butyl-2 [(carboxy-2' biphényl-4-yl) méthyl]-1 spirocyclopentane-4 imidazoline-2.

720 mg du produit obtenu à l'étape précédente sont traités par 10 ml de TFA dans 10 ml de DCM, pendant 4 heures, sous agitation à TA. Après concentration à sec, le résidu est trituré dans l'éther, la solution obtenue est concentrée et l'on obtient le produit attendu sous forme d'une mousse.
- Spectre RMN :
0,9 ppm : t : 3 H : CH₃(nBu)
1,4 ppm : sext: 2 H : CH₃-CH₂-CH₂-CH₂-
1,7 ppm : quint: 2 H : CH₃-CH₂-CH₂-CH₂-
1,8-2,5 ppm : m : 8 H: cyclopentane
2,75 ppm : t : 2 H : CH₃-CH₂-CH₂-CH₂-
4,8 ppm et 5 ppm : s : 2 H: -CH₂-C₆H₄-
6,6-7,8 ppm : m : 13 H : aromatiques

Le spectre RMN indique la présence des 2 formes cis et trans du méthylène.

### EXEMPLE 10

Trifluoroacétate de n-butyl-2 [(carboxy-2' biphényl-4-yl) méthyl]-1 (cyano-1 méthoxycarbonyl-1 méthylène)-5 spirocyclopentane-4 imidazoline-2.

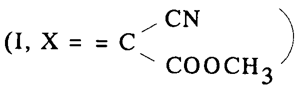

### A) n-butyl-2 (cyano-1 méthoxycarbonyl-1 méthylène)-5 spirocyclopentane-4 imidazoline-2

Ce composé est préparé en faisant agir le cyanoacétate de méthyle sur le composé préparé à l'exemple 4, étape A) et en suivant le procédé décrit à l'exemple 9, étape A).
- Spectre RMN :
0,9 ppm : t : 3 H : CH₃(nBu)
1,35 ppm : sext : 2 H : CH₃-CH₂-CH₂-CH₂-
1,6 ppm : quint : 2 H : CH₃-CH₂-CH₂-CH₂-
1,65-2,4 ppm : m : 8 H: cyclopentane
2,55 ppm : t : 2 H : CH₃-CH₂-CH₂-CH₂-
3,8 ppm : s : 3 H : OCH₃
11,4 ppm : s : 1 H : NH

### B) Trifluoroacétate de n-butyl-2[(carboxy-2' biphényl-4-yl) méthyl]-1 (cyano-1 méthoxycarbonyl-1 méthylène)-5 spirocyclopentane-4 imidazoline-2.

En opérant selon le procédé décrit à l'exemple 9, étapes B) et C), on prépare le composé attendu. Ce composé cristallise dans l'hexane additionné d'un peu d'éther.
Fc = 52-55°C
- Spectre RMN :
1,8 ppm : t : 3 H : CH₃(nBu)
1,2-2,6 ppm : m : 14 H cyclopentane + CH₃-CH₂-CH₂-CH₂-
3,6 ppm : d : 3 H : CH₃O-
5,2 et 5,4 ppm : 2 s : 2 H : -CH₂-C₆H₄-
6,8-7,7 ppm : m : 8 H : aromatiques

Le spectre RMN indique la présence de 2 formes cis et trans du méthylène substitué en équilibre.

### EXEMPLE 11

(benzyloxycarbonyl-1 cyano-1 méthylène)-5 n-butyl-2 [(carboxy-2' biphényl-4-yl) méthyl]-1 spirocyclopentane-4 imidazoline-2.

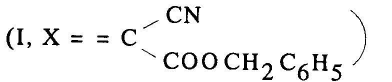

### A) (benzyloxycarbonyl-1 cyano-1 méthylène)-5 n-butyl-2 spirocyclopentane-4 imidazoline-2.

Ce composé est préparé en faisant agir le cyanoacétate de benzyle sur le composé préparé à l'exemple 4, étape A, et en suivant le procédé décrit à l'exemple 9, étape A. Le composé obtenu cristallise dans un mélange éther-hexane.
Fc = 91°C
- Spectre RMN :
0,8 ppm : t : 3 H : CH₃(nBu)
1,25 ppm : sext : 2 H : CH₃-CH₂-CH₂-CH₂-
1,5 ppm : quint : 2 H : CH₃-CH₂-CH₂-CH₂-
1,6-2,2 ppm : m : 8 H cyclopentane
5,15 ppm : s : 2 H : OCH₂-C₆H₅
11,4 ppm : s : 1 H : NH

### B) (benzyloxycarbonyl-1 cyano-1 méthylène)-5 n-butyl-2 [(carboxy-2' biphényl-4-yl) méthyl]-1 spirocyclopentane-4 imidazoline-2.

En opérant selon le procédé décrit à l'exemple 9, étapes B) et C), on obtient le composé attendu qui cristallise dans un mélange hexane-éther.
Fc = 92°C
- Spectre RMN :
0,75 ppm : q : 3 H : CH₃(nBu)
1,1-2,4 ppm : m : 14 H cyclopentane + CH₃-CH₂-CH₂-CH₂-
5 ppm: s dédoublé: 2 H: OCH₂-C₆H₅
5,2 et 5,4 ppm : 2 s : 2 H :-CH₂-C₆H₄-
6,8-7,65 ppm : m : 13 H : aromatiques

Le spectre RMN indique que les formes cis et trans du méthylène sont en équilibre.

### EXEMPLE 12

n-butyl-2[(carboxy-2' biphényl-4-yl) méthyl]-1 [cyano-1 (fur-2-ylcarbonyl)-1 méthylène]-5 spirocyclopentane-4 imidazoline-2.

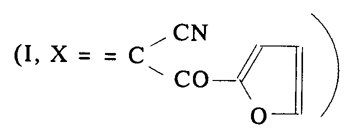

### A) n-butyl-2[cyano-1 (fur-2-ylcarbonyl)-1 méthylène]-5 spirocyclopentane-4 imidazoline-2.

Ce composé est préparé par addition du furoylacétonitrile sur le composé préparé à l'exemple 4, étape A) et selon le procédé décrit à l'exemple 9, étape A).
- Spectre RMN :
0,95 ppm : t : 3 H : CH₃(nBu)
1,4 ppm : sext : 2 H : CH₃-CH₂-CH₂-CH₂-
1,65 ppm : quint : 2 H : CH₃-CH₂-CH₂-CH₂-
1,7-2,5 ppm : m : 8 H : cyclopentane
2,6 ppm : t : 2 H : CH₃-CH₂-CH₂-CH₂-
6,8 ppm : q : 1 H : furyl
7,6 ppm : d : 1 H : furyl
8,1 ppm : d : 1 H : furyl
12,4 ppm : s : 1 H : NH

### B) n-butyl-2[(carboxy-2' biphényl-4-yl) méthyl]-1 [cyano-1 (fur-2-ylcarbonyl)-1 méthylène]-5 spirocyclopentane-4 imidazoline-2.

En opérant selon le procédé décrit à l'exemple 9, étapes B) et C), on obtient le composé attendu. Ce composé cristallise dans un mélange éther-hexane.
Fc = 159°C
- spectre de masse : MH⁺ = 522
- Spectre RMN
   0,8 ppm : t : 3 H : CH₃(nBu)
   1,3 ppm : sext : 2 H : CH₃-CH₂-CH₂-CH₂-
   1,6 ppm : quint : 2 H : CH₃-CH₂-CH₂-CH₂-
   1,7-2,4 ppm : m : 8 H : cyclopentane
   2,6 ppm : t : 2 H : CH₃-CH₂-CH₂-CH₂-
   4,9 ppm : s : 2 H : -CH₂-C₆H₄-
   6,4-7,8 ppm : m : 11 H : aromatiques + furyl

Le spectre RMN indique la présence d'une seule forme (cis ou trans) du méthylène substitué.

### EXEMPLE 13

n-butyl-2[(carboxy-2' biphényl-4-yl) méthyl]-1 [cyano-1 (thien-2-ylcarbonyl)-1 méthylène]-5 spirocyclopentane-4 imidazoline-2.

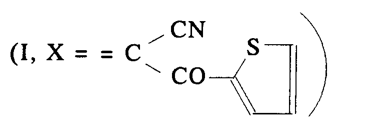

### A) n-butyl-2[cyano-1 (thien-2-ylcarbonyl)-1 méthylène]-5 spirocyclopentane-4 imidazoline-2.

Ce composé est préparé par action du thiénoylacétonitrile sur le composé préparé à l'exemple 4, étape A) et selon le procédé décrit à l'exemple 9, étape A). Le produit obtenu cristallise dans un mélange éther-hexane.
Fc = 86-89°C
- Spectre RMN :
0,85 ppm : t : 3 H : CH₃(nBu)
1,3 ppm : sext : 2 H : CH₃-CH₂-CH₂-CH₂-
1,55 ppm : quint : 2 H : CH₃-CH₂-CH₂-CH₂-
1,8-2,4 ppm : m : 8 H : cyclopentane + CH₃-CH₂-CH₂-CH₂-
7,2 ppm : q : 1 H : thiényl
7,95 ppm : q : 1 H : thiényl
8,15 ppm : d : 1 H : thiényl
12,3 ppm : s : 1H : NH

### B) n-butyl-2[(carboxy-2' biphényl-4-yl) méthyl]-1 [cyano-1 (thien-2-ylcarbonyl)-1 méthylène]-5 spirocyclopentane-4 imidazoline-2.

En opérant selon le procédé décrit à l'exemple 9, étapes B) et C), on prépare le composé attendu. Ce composé cristallise dans un mélange éther-hexane.
Fc = 162°C
- Spectre RMN :
0,9 ppm : t : 3 H : CH₃(nBu)
1,4 ppm : sext : 2 H : CH₃-CH₂-CH₂-CH₂-
1,65 ppm : quint : 2 H : CH₃-CH₂-CH₂-CH₂-
1,8-2,4 ppm : m : 8 H : cyclopentane
2,7 ppm : t : 2 H : CH₃-CH₂-CH₂-CH₂-
5 ppm : s : 2 H : -CH₂-C₆H₄-
6,6-8,0 ppm : m : 11 H : aromatiques + thienyl

Le spectre RMN indique la présence d'une seule forme (cis ou trans) du méthylène substitué.

### EXEMPLE 14

n-butyl-2[(cyano-2' biphényl-4-yl) méthyl]-1 [dicyano-1,1 méthylène]-5 spirocyclopentane-4 imidazoline-2.
et n-butyl-2 [cyano-1 (tétrazol-5-yl)-1 méthylène]-5 spirocyclopentane-4 [((tétrazol-5-yl)-2' biphényl-4-yl) méthyl]-1 imidazoline-2.

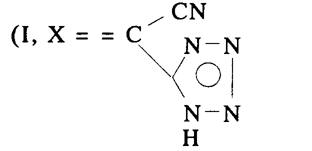

### A) n-butyl-2 (dicyano- 1,1 méthylène]-5 spirocyclopentane-4 imidazoline-2.

Ce composé est celui préparé à l'exemple 4, étape B). Il peut également être préparé en faisant agir le malonitrile sur le composé préparé à l'exemple 4, étape A et en suivant le procédé décrit à l'exemple 9, étape A). Le produit obtenu cristallise dans l'éther.
Fc = 145°C

### B) n-butyl-2 [(cyano-2' biphényl-4-yl) méthyl]-1 [dicyano-1,1 méthylène]-5 spirocyclopentane-4 imidazoline-2.

Dans 10 ml de DMF anhydre, on mélange 1,05 g du composé de l'étape A), 1,2 g de carbonate de potassium et 2,3 g de bromométhyl-4 cyano-2' biphényle (préparé selon la demande de brevet européen EP 324377). On chauffe à 70°C pendant 24 heures, sous agitation. On concentre à sec puis le résidu est extrait à l'acétate d'éthyle puis la phase organique est lavée à l'eau et par une solution saturée de chlorure de sodium. Le produit est purifié par chromatographie sur silice, en éluant par un mélange heptane-AcOEt (7/3, v/v).
- Spectre RMN :
0,8 ppm : t : 3 H: CH₃(nBu)
1,2 ppm : sext : 2 H : CH₃-CH₂-CH₂-CH₂-
1,5 ppm : quint : 2 H : CH₃-CH₂-CH₂-CH₂-
1,8-2,5 ppm : m : 10 H : cyclopentane + CH₃-CH₂-CH₂-CH₂-
5,35 ppm : s : 2 H : -CH₂-C₆H₄-
7,2-8,0 ppm : m : 8 H : aromatiques

### C) n-butyl-2[cyano-1 (tétrazol-5-yl)-1 méthylène]-5 spirocyclopentane-4 [((tétrazol-5-yl)-2' biphényl-4-yl) méthyl]-1 imidazoline-2.

On prépare un mélange contenant 1,58 g du composé préparé à l'étape précédente, 3 ml de chlorure de tributylétain et 760 mg d'azidure de sodium dans 15 ml de xylène et l'on chauffe à reflux pendant 60 heures. Le milieu réactionnel est repris par un mélange éther-soude à 5% (50/50, v/v) et laissé 15 minutes sous agitation à TA. Après décantation, la phase aqueuse est acidifiée à pH1 par addition d'acide chlorhydrique. On extrait la gomme formée par addition d'AcOEt. On concentre la phase organique, on reprend le résidu par de l'éther et on effectue une chromatographie sur silice en éluant par un mélange DCM/MeOH/ammoniaque à 20% d'eau (85/15/1,6, v/v/v). On obtient 1 g du produit attendu.
- Spectre de masse : MH⁺ : 520
- Spectre RMN :
   0,75 ppm : t : 3 H : CH₃(nBu)
   1,2 ppm : sext : 2 H : CH₃-CH₂-CH₂-CH₂-
   1,45 ppm : quint : 2 H : CH₃-CH₂-CH₂-CH₂-
   1,8-2,4 ppm : m : 10 H : cyclopentane + CH₃-CH₂-CH₂-CH₂-
   4,6 ppm : s : 2 H : -CH₂-C₆H₄-
   6,4-7,8 ppm : m : 8 H: aromatiques

Le spectre RMN indique la présence d'une forme (cis ou trans) du méthylène substitué.

### EXEMPLE 15

n-butyl-2[(carboxy-2' biphényl-4-yl) méthyl]-1 (cyano-1 phénylsulfonyl-1 méthylène)-5-spirocyclopentane-4 imidazoline-2.

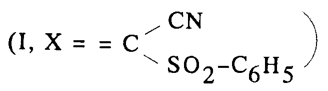

### A) n-butyl-2(cyano-1 phénylsulfonyl-1 méthylène]-5 spirocyclopentane-4 imidazoline-2.

Ce composé est préparé par action du phénylsulfonylacétonitrile sur le composé préparé à l'exemple 4, étape A), en suivant le procédé décrit à l'exemple 9, étape A).
- Spectre RMN :
0,85 ppm : t : 3 H : CH₃(nBu)
1,2 ppm : sext : 2 H : CH₃-CH₂-CH₂-CH₂-
1,6 ppm : quint : 2 H : CH₃-CH₂-CH₂-CH₂-
1,7-2,4 ppm : m : 8 H : cyclopentane
2,5 ppm : t : 2 H : CH₃-CH₂-CH₂-CH₂-
7,5-8 ppm : m : 5 H : aromatiques
5 ppm : s : 1 H : NH

### B) n-butyl-2[(carboxy-2' biphényl-4-yl) méthyl]-1 (cyano-1 phénylsulfonyl-1 méthylène)-5 spirocyclopentane-4 imidazoline-2.

En opérant selon le procédé décrit à l'exemple 9, étapes B et C, on prépare le composé attendu. Celui-ci cristallise dans un mélange éther-hexane.
Fc = 172°C
- Spectre de masse : MH⁺ : 568
- Spectre RMN :
   0,7 ppm : quint : 3 H : CH₃(nBu)
   1-1,4 ppm : m : 4 H : CH₃-CH₂-CH₂-CH₂-
   1,6-2,6 ppm : m : 10 H : cyclopentane + CH₃-CH₂-CH₂-CH₂-
   5,25 ppm : d : 2 H : -CH₂-C₆H₄-
   5,6-7,7 ppm : m : 13 H : aromatiques
   12,7 ppm : s : 1 H: CO₂H

Le spectre RMN indique la présence d'un mélange des formes cis et trans du méthylène en équilibre.

### EXEMPLE 16

Trifluoroacétate de n-butyl-2[cyano-1 (trifluorométhyl-3 benzoyl)-1 méthylène]-spirocyclopentane-4 [(carboxy-2' biphényl-4-yl) méthyl]-1 imidazoline-2.

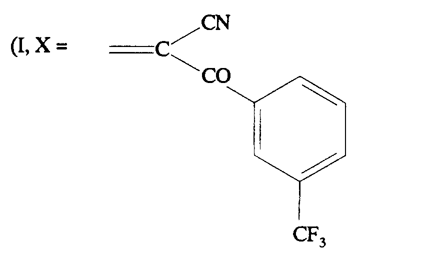

### A) n-butyl-2 [cyano-1 (trifluorométhyl-3 benzoyl)-1 méthylène]-5 spirocyclopentane-4 imidazoline-2.

Ce composé est préparé par action du (trifluorométhyl-3 benzoyl) acétonitrile sur le composé préparé à l'exemple 4, étape A) et selon le procédé décrit à l'exemple 9, étape A). Le produit obtenu est purifié par chromatographie sur silice en éluant par un mélange heptane-acétone (9/1, v/v).
Fc = 72-75°C
- Spectre RMN
0,8 ppm : t : 3 H : CH₃(nBu)
1,25 ppm : sext : 2 H : CH₂-CH₂-CH₂-CH₂-
1,55 ppm : quint : 2 H : CH₃-CH₂-CH₂-CH₂-
1,6-2,4 ppm : m : 8 H : cyclopentane
2,6 ppm : t : 2 H : CH₃-CH₂-CH₂-CH₂-
7,6-8 ppm : m : 4 H : aromatiques
- IR(KBr):2200 cm⁻¹

### B) Trifluoroacétate de n-butyl-2 [cyano-1 (trifluorométhyl-3 benzoyl)-1 méthylène]-5 spirocyclopentane-4 [(carboxy-2' biphényl-4-yl) méthyl]-1 imidazoline-2.

En opérant selon le procédé décrit à l'exemple 9, étapes B) et C), on a préparé le composé attendu. Ce composé cristallise dans un mélange éther-hexane.
Fc = 62-67°C
- Spectre RMN :
0,8 ppm : t : 3 H: CH₃(nBu)
1,35 ppm : sext : 2 H : CH₃-CH₂-CH₂-CH₂-
1,60 ppm : quint : 2 H :CH₃-CH₂-CH₂-CH₂-
1,80-2,4 ppm : m : 8 H : cyclopentane
2,65 ppm : t : 2 H : CH₃-CH₂-CH₂-CH₂-
5 ppm : s : 2 H : -CH₂-C₆H₄-
6,8-7,8 ppm : m : 12 H: aromatiques

## Revendications

1. Un composé de formule : 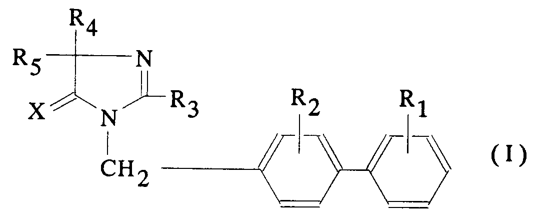 dans laquelle:
- R₁ et R₂ sont semblables ou différents et représentent chacun indépendamment l'hydrogène ou un groupe choisi parmi un alkyle en C₁-C₆, un alcoxy en C₁C₄ , un amino, un aminométhyle, un carboxy, un alcoxycarbonyle dans lequel l'alcoxy est en C₁-C₄, un cyano, un tétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol-1,3-yl-2) acétamide, un uréido, un cyano-2 guanidinocarbonyle, un cyano-2 guanidinométhyle, un imidazol-1-yl-carbonyle, un cyano-3 méthyl-2 isothioureidométhyle, à la condition qu'au moins l'un des substituants R₁ ou R₂ soit différent de l'hydrogène ;
- R₃ représente un hydrogène, un alkyle en C₁-C₆ non substitué ou substitué par un ou plusieurs atomes d'halogène, un alcényle en C₂-C₆, un cycloalkyle en C₃-C₇ , un phényle, un phénylalkyle dans lequel l'alkyle est en C₁-C₃ , un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un cycloalkyle en C₃-C₇, un phényle, un phénylalkyle dans lequel l'alkyle est en C₁-C₃, lesdits groupes alkyle, phényle ou phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄ , un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un un α-aminoacyle ou un groupe N-protecteur, ou R₄ et R₅ liés ensemble avec l'atome de carbone auquel ils sont liés constituent un indane ou un adamantane ;
- p + q = m;
- n est un nombre entier compris entre 2 et 11 ;
- m est un nombre entier compris entre 2 et 5 ;
- X représente un groupe N-R₇ ou un groupe C(CN) R₈ ;
- R₇ représente un alkyle en C₁-C₄ ou un groupe cyano ;
- R₈ représente l'hydrogène, un groupe cyano, un groupe tétrazolyle, un groupe *tert*-butyltétrazolyle, un groupe phénylsulfonyle ou un groupe COR₉ ;
- R₉ représente un thiényle, un furyle, un alcoxy en C₁-C₄, un phénoxy, un benzyloxy ou un phényle non substitué ou substitué par un halogène, un alkyle en C₁-C₄ ou un polyhalogénoalkyle en C₁-C₄, ;
et ses sels éventuels.

2. Un composé de formule (I) selon la revendication 1 dans lequel :
- R₁ est en position ortho et représente un carboxyle ou un tétrazolyle ;
- R₂ est l'hydrogène ;
- R₃ représente un alkyle en C₁-C₆ ;
- R₄ et R₅ liés ensemble avec le carbone auquel ils sont attachés, représentent un groupe de formule (CH₂)ₙ ;
- n est égal à 4 ou 5 ;
- X représente un groupe dicyanométhylène ;
et ses sels.

3. Un procédé pour la préparation d'un composé (I) selon la revendication 1 caractérisé en ce que :
a) on fait réagir un dérivé hétérocyclique de formule : 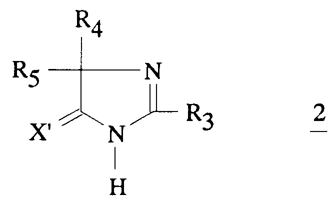 dans laquelle, R₃ , R₄, et R₅ ont les significations indiquées ci-dessus pour (I) et X' représente, soit un atome de soufre, soit X tel que défini dans la revendication 1, sur un dérivé du (biphényl-4-yl) méthyle de formule : 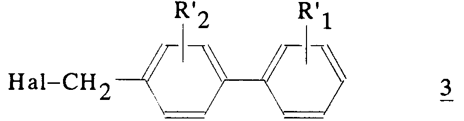 dans laquelle Hal représente un atome d'halogène et R'₁ et R'₂ représentent respectivement soit R₁ et R₂, soit un précurseur de R₁ et/ou R₂
b) éventuellement, le composé, ainsi obtenu de formule : 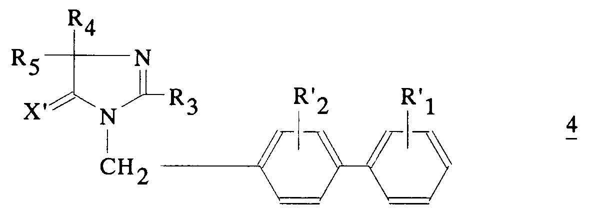 est traité pour préparer le composé (I) par transformation des groupes R'₁ et/ou R'₂ en respectivement R₁ et/ou R₂ et du groupe X' en X.
c) éventuellement, on transforme le composé ainsi obtenu en l'un de ses sels.

4. Un composé de formule : 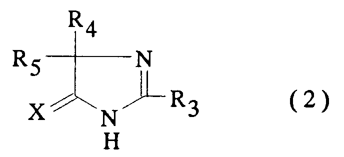 dans laquelle R₃, R₄, R₅ et X ont les valeurs données ci-dessus pour (I), dans la revendication 1.

5. Composition pharmaceutique contenant à titre de principe actif un composé selon l'une quelconque des revendications 1 ou 2.

6. Composition pharmaceutique contenant un composé selon l'une quelconque des revendications 1 ou 2 en association avec un composé bêtabloquant.

7. Composition pharmaceutique contenant un composé selon l'une quelconque des revendications 1 ou 2 en association avec un diurétique.

8. Composition pharmaceutique contenant un composé selon l'une quelconque des revendications 1 ou 2 en association avec un antiinflammatoire non stéroïdien.

9. Composition pharmaceutique contenant un composé selon l'une quelconque des revendications 1 ou 2 en association avec un antagoniste calcique.

10. Composition pharmaceutique contenant un composé selon l'une quelconque des revendications 1 ou 2 en association avec un tranquillisant.
